# EUROPEAN PATENT SPECIFICATION

(11) **EP 0 960 598 B1**
(45) Date of publication and mention of the grant of the patent: **16.07.2008**
(21) Application number: 98109811.4
(22) Date of filing: 28.05.1998
(51) Int. Cl.: A61B 5/00, A61B 5/022

(54) **A method and a device for noninvasive measurement of the blood pressure and for detection of arrhythmia**
Verfahren und Gerät zur nicht-invasiven Blutdruckmessung und Arrhythmienmessung
Procédé et dispositif de mesure non-invasive de la pression arterielle et mesure d'arythmie

(43) Date of publication of application: 01.12.1999
(73) Proprietor: Microlife Intellectual Property GmbH, 9443 Widnau (CH)
(72) Inventor: Forstner, Klaus, 71679 Asperg/Württemberg (DE)
(74) Representative: Hepp, Dieter

(56) References cited:
- EP-A- 0 841 034
- US-A- 4 356 827
- US-A- 5 680 867

## Description

The invention relates to a method and a device for the non-invasive measurement of the blood pressure and for simultaneous detection of arrhythmia.

The clinical standard of measuring the arterial blood pressure is the auscultation of audible sounds during the release of an inflated cuff in the cubital region of the brachial artery. The method is called the Riva/Rocci method by analysis of the so-called Korotkoff pulse sounds. First, non-invasive blood pressure measuring devices automatically recorded the Korotkoff sounds by the integration of a microphone into the cuff design. Although these devices proved to be very accurate, the critical microphone positioning and the occurrence of artefact sounds limited the clinical use of this method. Detection of arrhythmia proves to be an important parameter for prevention of cardiac diseases. It is known in the art to determine arrhythmic activities by means of an electrocardiographic method. Since both arrhythmia and chronic hypertension is associated with a severe risk in establishing cardiovascular diseases such as arteriosclerosis and cerebral ischaemia, it is known to simultaneously measure the blood pressure of the patient and to detect arrhythmic activities by the use of an electrocardiogram.

From US patent 4,262,674 it is known, to simultaneously measure the blood pressure based on Korotkoff sounds and to determine time differences between two consecutive pulses. The method according to this US patent is difficult to carry out because of the mentioned critical microphone positioning and the occurrence of artefact sounds. Further, the method according to the US patent is based on a strict consecutive treatment of measured pulses. The procedure does not analyse the total pulse pattern during the measuring time.

US-A-5 680 867 discloses a device for simultaneously measuring the blood pressure by non-invasive oscillographic method and detecting arrhythmia by analysing pulse time differences.

EP-A-0 841 034 shows a similar arrhythmia detector which performs a FFT or wavelet analysis of the plethysmographic signal.

The oscillometric non invasive measurement of the arterial blood pressure has been clinically introduced at the beginning of the 1980s. The technology required a pneumatic pressure pulse detecting system and a microcomputer based signal analysis. Due to the fast technological progress on semiconductors, the devices were consequently

equipped with smaller but more powerful microcomputers, programs and memories. In that way, the accuracy and stability of the non-invasive arterial blood pressure measurement was gradually improved.

The oscillometric method does not detect pulse sounds, but pressure changes within the cuff during the deflation process. The pressure pulses are generated by small amounts of streaming blood passing the vessels under the partially inflated cuff. Oscillometric devices avoid the use of a microphone and just require the application of a cuff at the patient's application site. For that reason, this technology was not only widely introduced clinically but was further integrated within a growing number of small blood pressure measuring devices, built for home use application.

Non-invasive oscillometric arterial blood pressure measuring units for home use were introduced widely towards the end of the 1980s. These devices measure and display three values: the arterial systolic pressure, the arterial diastolic pressure and finally the pulse frequency; all parameters are derived from the cuff application site.

The medical relevance of these devices is high. They are used in persons for whom regular measurements are recommended as a preventative measure, as well as in patients undergoing hypertensive or hypotensive blood pressure treatment. Since the age-dependent prevalence of hypertension is very high within the industrialised countries, regular blood pressure measurement serves as a crucial diagnosis measure to supplement the physician's diagnostic results.

As chronic hypertension is associated with a severe risk in establishing certain cardiovascular diseases, e.g. arteriosclerosis and cerebral ischaemia, a physiological adjustment of the arterial blood pressure - especially the diastolic arterial blood pressure - has been recognised as a highly efficient risk reduction of such disorders.

Further, a broad range of cardiac and extra-cardiac diseases are accompanied by rhythm disorders of the heart. This applies e.g. for diseases of the coronary arteries, diseases of the myocardial muscle, and disorders of the heart's excitation. In some cases, arrhythmia are significant early symptoms indicating the increased risk of developing certain severe heart malfunctions.

Some relevant arrhythmic heart activities are characterised by pathological variations of peripheral pressure pulse curves. So, pressure pulse curves may be used to identify certain arrhythmic events of the heart. Since pressure pulsations are monitored in the course of the oscillometric measurement, the analysis of the pulse pattern may be used to identify arrhythmic events. The analysis of the pulse rhythm is based on the analysis of pulse time difference, which is the time span between two correspondent pulse characteristics.

In case of a pathological rhythm status, pulse time differences may vary significantly in the course of the NIBP ("non-invasive blood pressure") measurement, or may be basically beyond physiological ranges. That means pulse time differences either change significantly or prove to be too high or too low. This is the basis of the detection of clinical relevant arrhythmia activities:
- Supraventricular premature contractions: variation high due to pulse loss
- Ventricular premature contractions: variation high due to pulse loss
- Atrial fibrillation variation high due to absolute arrhythmia
- Paroxysmal supraventricular tachycardia mean time difference low
- Sinus - Tachycardia mean time difference low
- Sinus - Bradycardia mean time difference high
- Ventricular Bradycardia mean time difference high

Some arrhythmic activities are unfortunately not noticed by patients, since the associated symptoms remain minimal. However, certain types indicate a severe forthcoming heart malfunction. For this reason, early diagnosis and therapy may diminish the risk of serious complications.

Arrhythmia are very common within the population of industrialised countries. For example about 0.5% of all adults, 3% of all people aged over 60, and 15% of all people over 70 suffer from atrial fibrillation.

It is an object of the present invention to overcome the drawbacks of the prior art, especially to provide a device and a method for simultaneously measuring blood pressure and detecting arrhythmic activities, which are both reliable and easy to apply, which are adapted for home use and which are easy to manufacture and carry out.

According to the present invention, these objects are solved with a method and a device according to the independent patent claims.

According to the invention, a pulse distribution is measured and is analysed by the device. The pulse distribution is investigated by using known medical criteria representing physiological or pathophysiological conditions, which are stored within the device. These criteria decide whether there is reason to diagnose an arrhythmic activity in the course of the NIBP measurement. Transformation, calculation and comparison can be effected analogically, digitally or by direct EDP comparison of curves. There can be signal storage and batchwise processing or on-line processing.

According to the method of the present invention, the blood pressure is preferably measured by measuring the pressure pulses of the blood in an artery by oscillometric measurement techniques. Measurement and analysis of the pressure pulses by oscillometric techniques allows the calculation of the blood pressure, especially the systolic and diastolic pressures. In order to indicate an arrythmia, all values of the time difference between consecutive pulses are determined. The values of a plurality of consecutive pulse time differences are stored in a storing unit. The distribution of the pulse time differences is subsequently calculated in a calculating unit. In order to determine the existence of arrhythmic activities, the values and the distribution of the pulse time differences are compared with predetermined reference values and predetermined reference distributions of the pulse time differences in a comparing unit. In order to indicate the existence of arrhythmic activities, the comparing unit is caused to generate a signal if the values and/or distributions of the pulse time differences on the one hand and the predetermined arrhythmic specific values and distributions on the other hand are sufficiently close to each other. The signal can be optical, acoustic or any other kind of signal.

The method according to the invention is intended for a broad range of medical use. This includes the clinical application, the emergency based application, as well as home use. The indication of existence of arrhythmic activities tells the patient to consult a physician for a more detailed cardiac diagnosis.

The pressure pulse signals acquired by the oscillometric measurement are preferably amplified in a pulse pressure amplifier which generates an amplified signal. The amplified signal can subsequently be filtered and converted into a digital signal with an analogue/digital converter. Amplification and A/D conversion is known to those skilled in the art and is not the object of the present invention.

In a preferred embodiment, a pulse time difference histogram is generated in the calculating unit. Representation of the pulse time difference distribution in a histogram allows an especially reliable and precise detection of the existence of arrhythmic activities.

The device according to the present invention substantially comprises a blood pressure monitor for indicating the blood pressure values and a cuff for the non-invasive oscillographic measurement of the blood pressure. The cuff is connected to the blood pressure monitor. A time difference measuring unit is used to determine the values of time pulse differences between two consecutive pulses. The device further comprises a storing unit for storing a plurality of consecutive time pulse differences which have been determined by the time difference measuring unit. The device is further provided with a calculating unit for calculating a statistic distribution of the time pulse differences and a comparing unit for comparing the values and the statistic distribution of the time pulse differences with predetermined reference values and reference distributions. The comparing unit is adapted to create a signal when the value and/or the distribution of the time pulse differences on the one hand and the predetermined value or reference distribution representing pathophysiological conditions on the other hand are sufficiently close to each other. It is also conceivable to create a signal if the difference between the measured parameters and criteria representing physiological values exceeds a certain amount.

The device comprises preferably a pulse pressure amplifier which creates an amplified pulse signal. The device further comprises a signal filter and optionally an A/D converter which filters and converts the amplified signal into a digital signal. The calculating unit is adapted to calculate a pulse time difference histogram. Such calculating units are known to those skilled in the art and are not the object of the present invention.

The device is preferably provided with indication means for indicating the existence of arrhythmia. Any kind of known indication means are possible - preferably, for instance, signal lamps, LEDs or an LCD display. It is also conceivable to provide acoustic indication means.

The apparatus detects arrhythmic activities during the non-invasive measurement of the arterial blood pressure. The apparatus uses a cuff which is wrapped around the patient's upper leg, lower leg, toe, upper arm, forearm or finger. After inflation of the cuff, the pressure is released gradually in order to measure arterial pulses by detecting pressure pulses. An inbuilt memory stores the pulse amplitudes of each pulse, the associated cuff pressure and the time differences between two consecutive pulses. While the first and second parameters are used to determine the systolic, mean and diastolic arterial blood pressure, the third parameter is applied in order to detect arrhythmic pulse activities of the patient. The detection includes premature ventricular and supraventricular contractions, absolute arrhythmia as well as tachycardia and bradycardia. The apparatus displays the result of the rhythmic analysis and indicates the user to take actions according to the result.

The analysis of the pulse time difference within a oscillometric measurement discloses the mean pulse time difference and the scattering of pulse time differences within one complete measurement cycle. Therefore the pulse time differences are investigated for every full measurement in order to detect possible arrhythmic activities.

The present invention analyses the pulse time difference signals derived from pulses within the pulse oscillogram. The analysis result is displayed beside the conventional values.

The invention will be more clearly understood with reference to the following figures, which show:
- Figure 1: a schematic view of a device according to the invention;
- Figure 2: a histogram of pulse time differences representing a standard physiological distribution;
- Figure 3: a pulse time difference histogram representing tachycardia;
- Figure 4: a pulse time difference histogram representing bradycardia;
- Figure 5: a pulse time difference histogram representing missing pulses; and
- Figure 6: a pulse time difference histogram representing absolute arrhythmia.
- Figure 7: shows the general principle of the invention

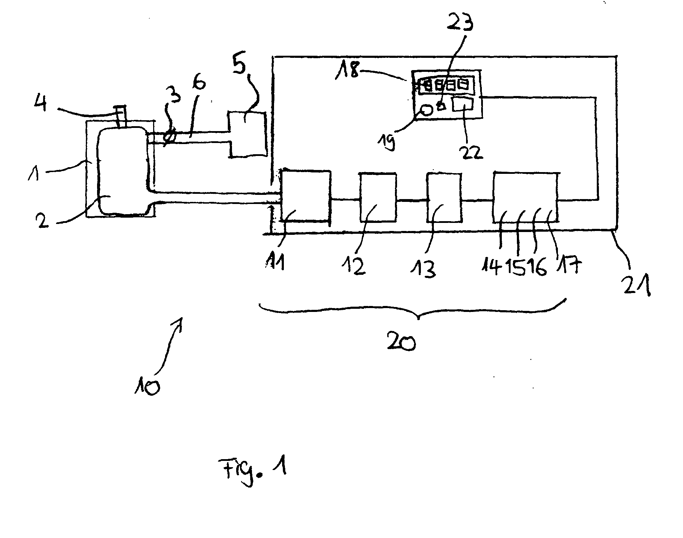

The oscillometric arterial pulse signals are derived from a non-invasive blood pressure measuring device 10, shown in Figure 1.

In order to sufficiently amplify the measured pulse signals, the device 10 is equipped with a pulse pressure amplifier 11. In that way, the influence of various noise signals is reduced and weak pulse signals can be identified. Within this stage, signals which do not fulfil the criteria of a valid pulse are suppressed. For example, pulses which are too weak or which are increasing/decreasing too fast are suppressed as they are due to movement artefacts.

The low noise amplified signal is filtered in a signal filter 12 and consequently converted into a digital signal by means of an A/D converter 13.

The digital signal provided by the A/D converter 13 is subsequently used to determine the pulse time differences between two consecutive pulses in a time pulse difference measuring unit 14. The values of the time pulse differences are stored in a storing unit 15. A calculating unit 16 is provided for calculating a statistic distribution of the time pulse differences. This distribution and the value of the pulse time differences are subsequently analysed by using reference distribution and predetermined reference values within a comparing unit 17. If the measured distribution is close to a stored distribution representing a pathophysiological condition, an arrhythmia indicating signal will be generated. If the measured distribution is close to a stored physiological distribution, no signal will be generated.

The pulse amplifier 11, filter 12, A/D converter 13, time pulse difference measuring unit 14, storing unit 15, calculating unit 16 and comparing unit 17 together may form a signal processing unit 20. The components of the signal processing unit 20 are known to those skilled in the art and need not be explained in more detail.

The signal provided by the signal processing unit 20 is coupled to a blood pressure monitor 21. The blood pressure monitor 21 is provided with a first indication field 18 for indicating the blood pressure values, with a second indication field 23 concerning the pulse frequency display, and with a third indication field 19 for indicating existence of arrhythmic activities. The first indication field 18 consists of a LED or LCD display and the second indication field 19 consists of a signal lamp or signal LED which is activated if the comparing unit detects existence of arrhythmic activities.

Further the instrument is equipped with user keys 22, an optional electronically regulated pump 5 and a pneumatic system 6. The latter comprises a cuff 1 with a bladder 2, a possibly automatically controlled release valve 3 and a fast switching valve 4, which is actively used in order to instantly release the system pressure from cuff 1.

The pressure measurement, A/D conversion and the signal amplification may be performed by using several technology methods, e.g. pressure to frequency conversion, direct pressure to voltage conversion (Piezoelectric element) or any further known method.

The arrhythmia detection system may be implemented by using hardware and software system components. The arrhythmia detection is based on the analysis of pulse time differences. Pulse time difference are referred to as T_{P}. The reason for changes of pulse time differences are twofold:
1. Pulse time differences T_{P} are constantly not in the physiological range.
2. The variation of pulse time differences T_{P} is too high.

The judgement of the measurement's pulse time differences T_{P} is performed by analyzing a pulse time difference histogram in the comparing unit 17, which is created during the measurement of the oscillometric pulses in the course of the non-invasive blood pressure measurement in the calculating unit 16.

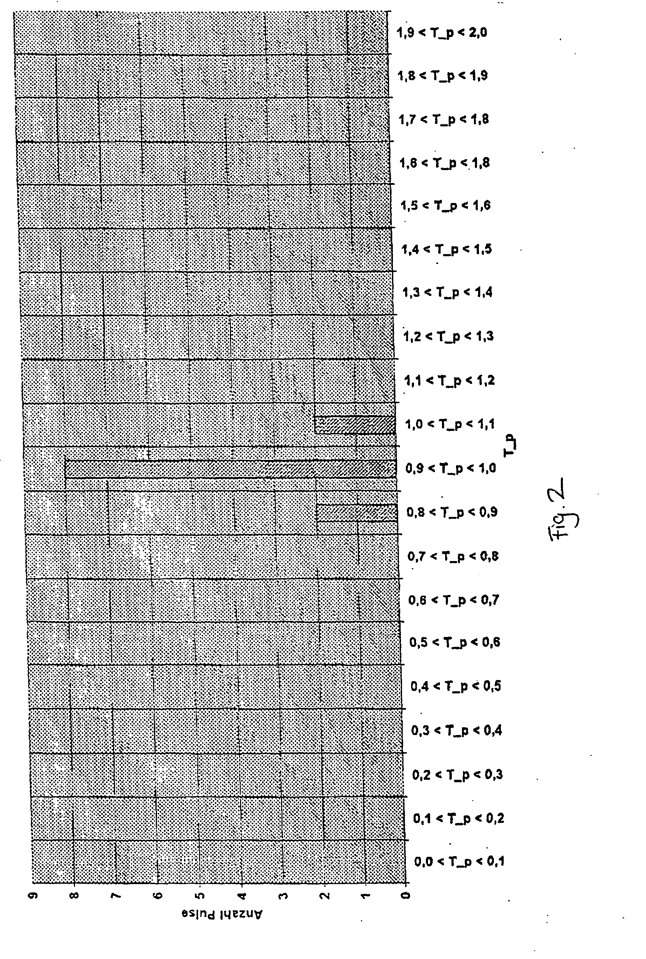

An example of a physiological pulse oscillogram is shown in Figure 2. The number of pulses is distributed around pulse time differences T_{P} between 0.8 and 1.1 seconds. A maximum is to be found between 0.9 and 1.0 seconds. This physiological distribution represents the reference distribution around a reference value of about 0.9 to 1.0 seconds. Both the scale of the pulse time differences ad the number of pulses represent an example.

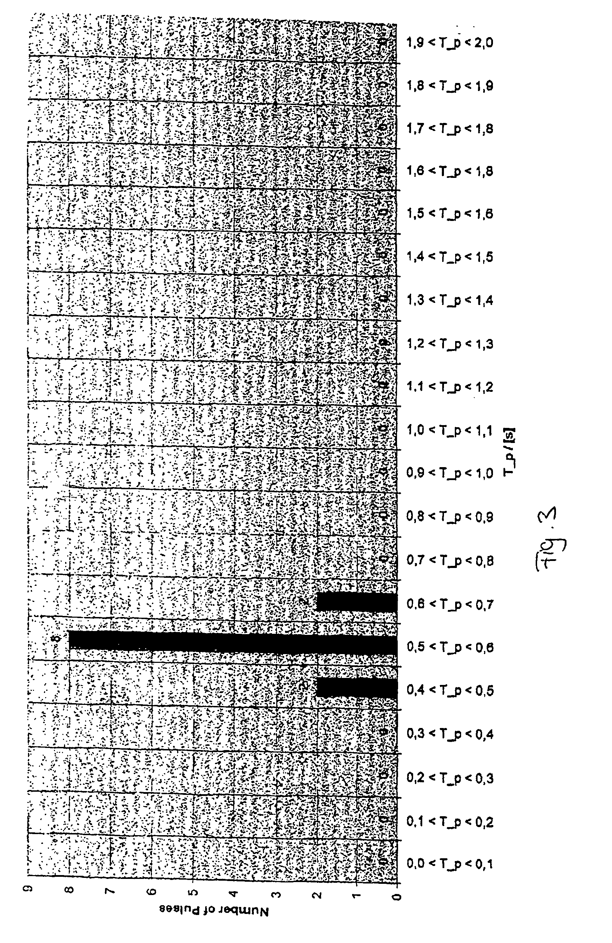

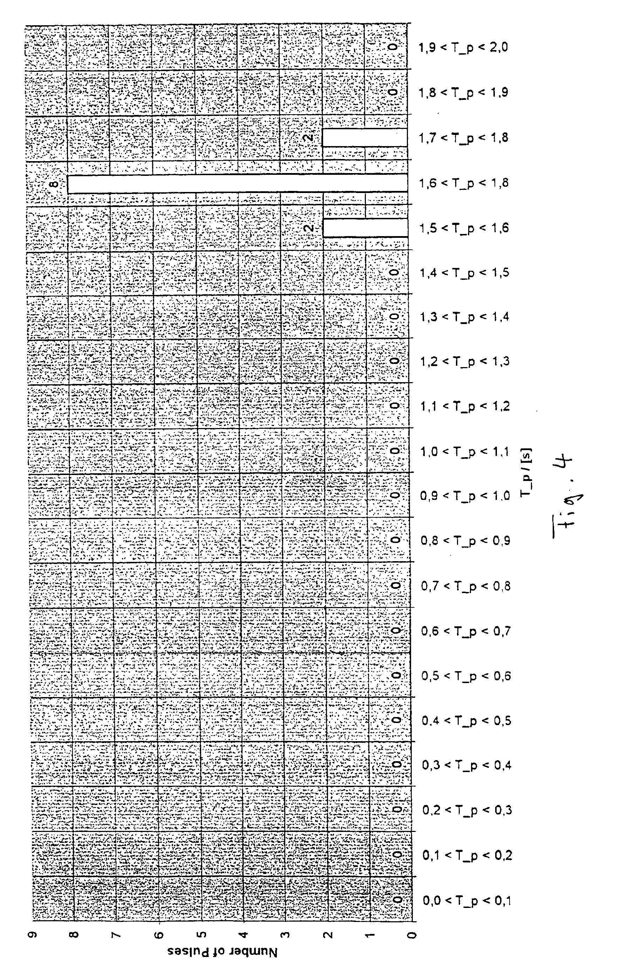

In Figures 3 and 4, pulse time differences are beyond the physiological limits. In Figure 3 the centre pulse frequency exceeds 100 beats per minute (bpm), which clinically fulfils the criteria of tachycardia if the patient's frequency was investigated under rest conditions.

In Figure 4, the pulse difference time histogram plots an example for a bradycardia. The centre frequency is smaller than 50 bpm.

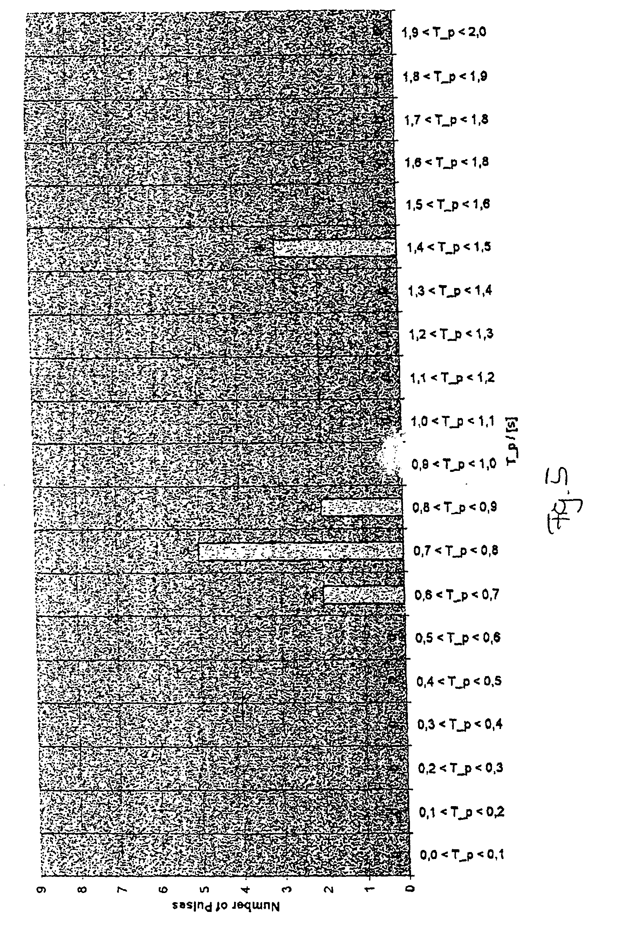

In Figure 5 pulses are missing or cancelled by the filter criteria of the system (invalid pulse exclusion). This effect leads to the registration of doubled pulse time differences, which can be clearly seen from the pulse time difference registration. There are two peaks within the pulse time difference histogram. In the example given, there is a first histogram peak at time difference of about 0.75 seconds, which is equal to 80 bpm. Further, there is another peak at 1.5 seconds, which means about 40 bpm. This latter frequency is artificial and is a consequence of missing pulses. By detecting a second peak at the doubled value of the base pulse frequency, missing pulses can be identified.

Missing pulses are clinically relevant as well as a frequent symptom. In particular, premature ventricular and supraventricular contractions are sources of missing pulses.

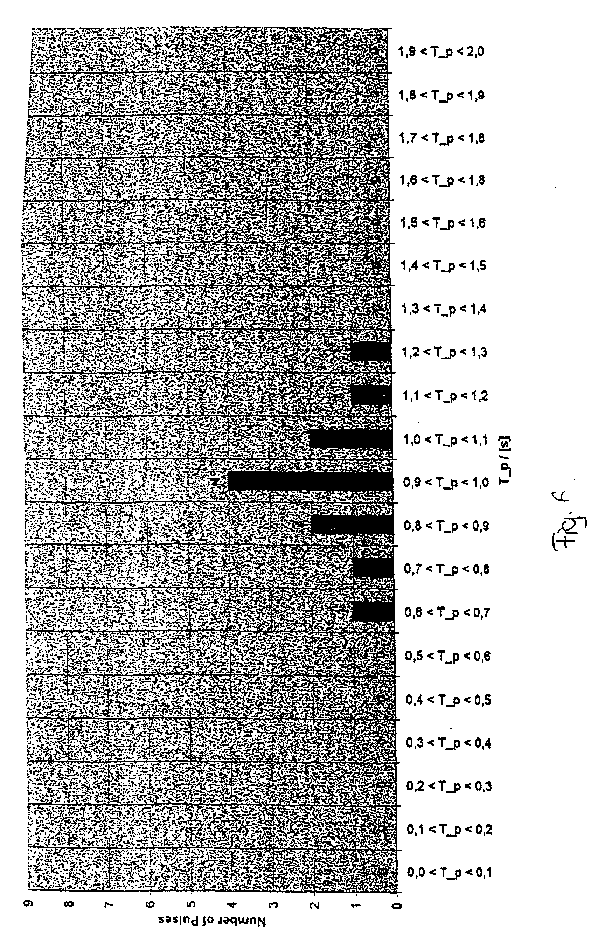

In Figure 6 the pulse difference times vary widely. Within the example the pulse frequency changes continuously from 45 bpm to about 95 bpm. This high degree of variation is related to a so called absolute arrhythmia. This kind of an arrhythmia might be caused by atrial fibrillation. Atrial fibrillation is a frequent clinical symptom which has to be treated normally.

The pulse distribution within the pulse time difference histogram is applied in order to investigate arrhythmic actions within the pulse oscillogram.

The comparing unit 17 compares the distribution of the pulse time differences according to the example histograms of Figures 3 to 6 and the values of the pulse time differences with certain criteria which are related reference histograms. If a shift towards short pulse time differences (as in Figure 3) is detected and the clinical criteria for tachycardia is fulfilled, the indication lamp 19 is activated and indicates existence of arrhythmic activities.

If the histogram is shifted towards high pulse differences as in Figure 4 and the clinical criteria for bradycardia is fulfilled, the signal lamp 19 is also activated.

The signal lamp 19 is also activated if missing pulses as in Figure 5 are detected or if the distribution is broadened due to an absolute arrhythmia as shown in Figure 6. It is also conceivable to activate different signal lamps depending on the type of arrhythmia disclosed in one of the Figures 3 to 6. All these indications are derived according to known clinical criteria representing pathophysiological and/or physiological conditions, which are stored within the system.

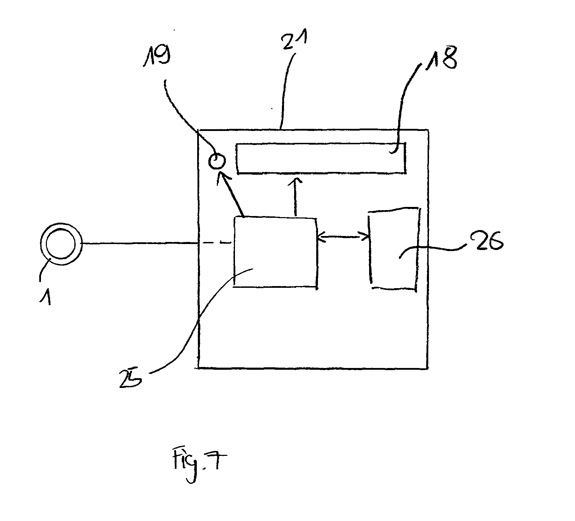

Figure 7 discloses the general principle of the invention. Blood pressure pulses are oscillographically measured with a cuff 1, coupled into a CPU 25 and transformed into pulse signals. The pulse signals area treated in the CPU 25, temporarily stored in a memory 26 and compared with predetermined pulse signals.

The blood pressure and pulse frequency are displayed on a display 18 and a signal is activated if the difference between the measured pulse signal and predetermined pulse signals exceed a certain amount.

The system may further comprise a unit to determine the systolic and arterial blood pressure as well as the pulse frequency by analysing the pulse oscillogram. This unit also displays the measurement results and is controlled by the user.

## Claims

1. A method for non-invasive measurement of blood pressure and for detection of arrhythmia, comprising the steps of
- measuring the pressure pulses of the blood in an artery by oscillometric measurement
- determining the value of the pulse time differences (Tₚ) between a plurality of consecutive pulses
- storing the values of a plurality of consecutive pulse time differences (Tₚ) in a storing unit (15)
- calculating a statistic distribution of the said pulse time differences (Tₚ) in a calculating unit (16)
- comparing the value and the statistic distribution of the pulse time differences (Tₚ) with predetermined physiological and/or pathophysiological values and distributions of pulse time differences (Tₚ) in a comparing unit (17)
- causing the comparing unit to generate a signal if the difference between the value and the statistic distribution of the pulse time differences (Tₚ) on the one hand and the predetermined physiological values and statistic distributions on the other hand exceed a predetermined amount,
and/or if the difference between the value and the statistic distribution of the pulse time difference on the one hand and the predetermined pathophysiological values and statistic distributions on the other hand is less than a predetermined amount.

2. A method according to claim 1, wherein the pressure pulse signals are amplified in a pulse pressure amplifier (11), generating an amplified signal, whereby invalid pulses are excluded according to pulse acceptance criteria integrated in the pulse pressure amplifier.

3. A method according to claim 2, wherein the amplified signal is filtered and converted into a digital signal with an A/D converter (13).

4. A method according to one of the claims 1 to 3, wherein a pulse time difference histogram is generated in the calculating unit (16).

5. A device (10) for simultaneously measuring the blood pressure and detecting arrhythmia, comprising
a blood pressure monitor (21)
a cuff (1) for non-invasive oscillographic measurement of the blood pressure, connected to said blood pressure monitor (21)
a time difference measuring unit (14) for determining the values of pulse time differences (Tₚ) between two subsequent pulses
a storing unit (15) for storing a plurality of subsequent pulse time differences (Tₚ) determined by said blood pressure monitor (21)
a calculating unit (16) for calculating a statistic distribution of the said pulse time differences (Tₚ)
a comparing unit (17) for comparing the said values and the said statistic distribution of pulse time differences (Tₚ) with predetermined reference values and distribution
said comparing unit (17) being adapted to create a signal when the difference between the said value and the said statistic distribution of the pulse time differences (Tₚ) on the one hand and the predetermined reference values and distribution on the other hand exceed a predetermined amount.

6. A device according to claim 5, wherein the device comprises a pulse pressure amplifier (11).

7. A device according to claim 5 or 6, wherein the device comprises a signal filter (12) and an A/D converter (13).

8. A device according to one of the claims 5 to 7, wherein the calculating unit (16) is adapted to calculate a pulse time difference histogram

9. A device according to one of the claims 5 to 8, wherein the device (10) comprises indication means (19) for indicating the existence of arrhythmia.

10. A device according to claim 9, wherein the indication means consist of at least one optical display (19).

## Patentansprüche

1. Verfahren für die nicht-invasive Blutdruckmessung und für die Erkennung von Arrhythmie, das folgende Schritte umfasst:
- Messung der Druckpulse des Blutes in einer Arterie durch oszillometrische Messung
- Bestimmung des Wertes der Pulszeitunterschiede (Tp) zwischen einer Vielzahl von aufeinanderfolgenden Pulsen
- Speicherung der Werte einer Vielzahl von aufeinanderfolgenden Pulszeitunterschieden (Tₚ) in einer Speichereinheit (15)
- Berechnung einer statistischen Verteilung der Pulszeitunterschiede (Tₚ) in einer Recheneinheit (16)
- Vergleich des Wertes und der statistischen Verteilung der Pulszeitunterschiede (Tp) mit vorbestimmten physiologischen und/oder pathophysiologischen Werten und Verteilungen von Pulszeitunterschieden (Tp) in einer Vergleichseinheit (17)
- Veranlassung, dass die Vergleichseinheit ein Signal erzeugt, wenn der Unterschied zwischen dem Wert und der statistischen Verteilung der Pulszeitunterschiede (Tp) einerseits und den vorbestimmten physiologischen Werten und statistischen Verteilungen andererseits einen vorbestimmten Betrag überschreitet,
und/oder wenn der Unterschied zwischen dem Wert und der statistischen Verteilung des Pulszeitunterschieds einerseits und den vorbestimmten pathophysiologischen Werten und statistischen Verteilungen andererseits kleiner ist als ein vorbestimmter Betrag.

2. Verfahren nach Anspruch 1, worin die Druckpulssignale in einem Pulsdruckverstärker (11) verstärkt werden, so dass ein verstärktes Signal erzeugt wird, wobei ungültige Pulse gemäß Pulsakzeptanzkriterien, die im Pulsdruckverstärker integriert sind, ausgeschlossen werden.

3. Verfahren nach Anspruch 2, worin das verstärkte Signal gefiltert und mit einem A/D-Wandler (13) in ein digitales Signal umgewandelt wird.

4. Verfahren nach einem der Ansprüche 1 bis 3, worin ein Pulszeitunterschieds-Histogramm in der Recheneinheit (16) erzeugt wird.

5. Gerät (10) zur gleichzeitigen Messung des Blutdrucks und zum Erkennen von Arrhythmie, umfassend
- ein Blutdrucküberwachungsgerät (21)
- eine Manschette (1) zur nicht-invasiven oszillografischen Messung des Blutdrucks, die mit dem Blutdrucküberwachungsgerät (21) verbunden ist
- eine Zeitunterschiedsmesseinheit (14) zur Bestimmung der Werte der Pulszeitunterschiede (Tp) zwischen zwei aufeinanderfolgenden Pulsen
- eine Speichereinheit (15) zum Speichern aufeinanderfolgender Pulszeitunterschiede (Tp), die vom Blutdrucküberwachungsgerät (21) bestimmt werden
- eine Recheneinheit (16) zur Berechnung einer statistischen Verteilung der Pulszeitunterschiede (Tₚ)
- eine Vergleichseinheit (17) zum Vergleichen der Werte und der statistischen Verteilung der Pulszeitunterschiede (Tp) mit vorbestimmten Referenzwerten und einer vorbestimmten Verteilung,
wobei das Vergleichsgerät (17) ein Signal erzeugt, wenn der Unterschied zwischen dem Wert und der statistischen Verteilung der Pulszeitunterschiede (Tp) einerseits und den vorbestimmten Referenzwerten und Verteilungen andererseits einen vorbestimmten Betrag übersteigt.

6. Vorrichtung nach Anspruch 5, worin die Vorrichtung einen Pulsdruckverstärker (11) umfasst.

7. Vorrichtung nach Anspruch 5 oder 6, worin die Vorrichtung einen Signalfilter (12) und einen A/D-Wandler (13) umfasst.

8. Vorrichtung nach einem der Ansprüche 5 bis 7, worin die Recheneinheit (16) ein Pulszeitunterschieds-Histogramm berechnen kann.

9. Vorrichtung nach einem der Ansprüche 5 bis 8, worin die Vorrichtung (10) Anzeigemittel (19) zum Anzeigen des Vorhandenseins von Arrhythmie umfasst.

10. Vorrichtung nach Anspruch 9, worin das Anzeigemittel mindestens eine optische Anzeige (19) aufweist.

## Revendications

1. Procédé de mesure non-invasive de la pression artérielle en vue de la détection de l'arythmie, lequel procédé comprend les étapes qui consistent à :
- mesurer les pulsations de pression du sang dans une artère par une mesure oscillométrique,
- déterminer la valeur des différences (Tₚ) de durée de pulsation entre plusieurs pulsations consécutives,
- mémoriser les valeurs de plusieurs différences (Tp) de durée de pulsations consécutives dans une unité de mémoire (15),
- dans une unité de calcul (16), calculer une distribution statistique desdites différences (Tp) de durée de pulsation,
- dans une unité de comparaison (17), comparer la valeur et la distribution statistique des différences (Tp) des durées de pulsation à une valeur physiologique prédéterminée et/ou à une valeur pathophysiologique prédéterminée et des distributions de différences (Tp) de durée de pulsation,
- faire délivrer par l'unité de comparaison un signal si les différences entre la valeur et la distribution statistique des différences (Tp) de durée de pulsation d'un côté et les valeurs physiologiques prédéterminées et les distributions statistiques prédéterminées d'un autre côté dépassent une quantité prédéterminée
et/ou si la différence entre la valeur et la distribution statistique des différences de durée de pulsation d'un côté et les valeurs pathophysiologiques prédéterminées et les distributions statistiques d'un autre côté sont inférieures à une quantité prédéterminée.

2. Procédé selon la revendication 1, dans lequel les signaux de pulsation de la pression sont amplifiés dans un amplificateur (11) de pression de pulsation qui délivre un signal amplifié au moyen duquel les pulsations non valides sont exclues selon un critère d'acceptation des pulsations intégré dans l'amplificateur de pression de pulsation.

3. Procédé selon la revendication 2, dans lequel le signal amplifié est filtré et converti en un signal numérique avec un convertisseur analogique-numérique (13).

4. Procédé selon l'une quelconque des revendications 1 à 3, dans lequel un histogramme des différences de durée de pulsation est produit dans l'unité de calcul (16).

5. Dispositif (10) pour mesurer simultanément la pression artérielle et détecter l'arythmie comprenant :
un dispositif de surveillance (21) de la pression artérielle,
un brassard (1) qui permet d'effectuer une mesure oscillographique non-invasive de la pression artérielle et qui est relié audit dispositif de surveillance (21) de la pression artérielle,
une unité (14) de mesure de la différence de temps qui détermine les valeurs des différences (Tp) de durée de pulsation entre deux pulsations qui se suivent,
une unité de mémoire (15) qui mémorise plusieurs différences (Tp) de durée de pulsation successives déterminées par ledit dispositif de surveillance (21) de la pression artérielle,
une unité de calcul (16) qui calcule une distribution statistique desdites différences (Tp) de durée de pulsation,
une unité de comparaison (17) qui compare lesdites valeurs et ladite distribution statistique des différences (Tp) de durée de pulsation à des valeurs de référence prédéterminées et à une distribution de référence prédéterminée,
ladite unité de comparaison (17) étant conçue pour délivrer un signal si les différences entre ladite valeur et ladite distribution statistique des différences (Tp) de durée de pulsation d'un côté et les valeurs de référence prédéterminées et les distributions d'un autre côté dépassent une quantité prédéterminée.

6. Dispositif selon la revendication 5, dans lequel le dispositif comprend un amplificateur (11) de pression de pulsation.

7. Dispositif selon la revendication 5 ou 6, dans lequel le dispositif comprend un filtre de signal (12) et un convertisseur analogique-numérique (13).

8. Dispositif selon l'une quelconque des revendications 5 à 7, dans lequel l'unité de calcul (16) est conçue pour calculer un histogramme des différences de durées de pulsation.

9. Dispositif selon l'une des revendications 5 à 8, dans lequel le dispositif (10) comprend des moyens d'indication (19) qui indiquent la présence d'une arythmie.

10. Dispositif selon la revendication 9, dans lequel les moyens d'indication consistent en au moins un dispositif d'affichage optique (19).
